# EUROPEAN PATENT APPLICATION

(11) **EP 4 295 768 A1**
(43) Date of publication of application: **27.12.2023**
(21) Application number: 22756119.8
(22) Date of filing: 14.02.2022
(51) Int. Cl.: A61B 5/1455

(54) **BLOOD SUGAR MEASURING INSTRUMENT**

(30) Priority: 19.02.2021 JP 2021025482
(71) Applicant: Zeon Corporation, Tokyo 100-8246 (JP); Tohoku University, Sendai-shi, Miyagi 980-8577 (JP); FURUKAWA ELECTRIC CO., LTD., Chiyoda-ku Tokyo 100-8322 (JP)
(72) Inventor: KAWAJIRI, Koji, Tokyo 100-8246 (JP); KURODA, Rihito, Sendai-shi, Miyagi 980-8577 (JP); FUJIHARA, Yasuyuki, Sendai-shi, Miyagi 980-8577 (JP); NAKAYAMA, Shota, Sendai-shi, Miyagi 980-8577 (JP); HASEGAWA, Hideaki, Tokyo 100-8322 (JP)
(74) Representative: Maiwald GmbH
(86) International application number: PCT/JP2022/005643
(87) International publication number: WO 2022/176801

(57) **Abstract**

To provide a blood glucose measurement device capable of measuring a blood glucose in the blood while suppressing the influence of light absorption by water in a living body, the blood glucose measurement device 10 comprises: a light source 11 that irradiates light having a wavelength selected from a wavelength band of 800 to 950 nm; a sensor 13 that receives light transmitted, reflected, or scattered in a living body and outputs information according to the amount of light received; and a blood glucose acquisition unit 14a that acquires the blood glucose in the blood in the living body based on the information obtained by the sensor 13.

## Description

### [FIELD OF THE INVENTION]

The present invention relates to a blood glucose measurement device capable of non-invasively measuring a glucose concentration in the blood (blood glucose).

### [BACKGROUND ART]

Conventionally, the development of a technology for non-invasively measuring the blood glucose in the living body without collecting blood, in particular, a technology for measuring the blood glucose in the living body using light has been promoted.

For example, Patent Document 1 below, describes a biological information acquisition / analysis device capable of improving the calculation accuracy of the blood glucose at the blood vessel site, by identifying a region where the intensity of the emitted light from the living body obtained by irradiating light of the first wavelength exceeds a predetermined threshold as a blood vessel site, using light of a first wavelength, having a large absorption with respect to the hemoglobin, which is a component peculiar to blood, and by separating the data based on the intensity of the outgoing light from the living body obtained by irradiating the light of the second wavelength between the region specified as the blood vessel site and the other region, using light of a second wavelength, having a large absorption with respect to the glucose. The biometric information acquisition / analysis device identifies a blood vessel site using a wavelength of 800 to 900 nm or 810 to 940 nm, as wavelength A having a large absorption with respect to the hemoglobin and the biometric information acquisition / analysis device obtains data including glucose intensity data only from the blood vessel site using wavelength of 1500 to 1700 nm as a wavelength B having a large absorption of glucose.

Further, for example, in Non-Patent Document 1 and Non-Patent Document 2 below describe a non-invasive blood glucose measurement using a near-infrared high-sensitivity CMOS (Complementary Metal Oxide Semiconductor) image sensor, as a means to detect the blood component concentration of the living body with high accuracy. In Non-Patent Document 1 and Non-Patent Document 2, experiments in which the glucose solution was dropped, convected, and diffused in the physiological saline in the cell was imaged by irradiating with light having a wavelength of 1050 nm, which is one of the absorption peaks of glucose, so that it is suggested that this image be used to determine glucose concentration.

### [CITATION LIST]

### [PATENT LITERATURE]

[Patent Document 1] Japanese Patent Publication No. 5623540

### [NON-PATENT LITERATURE]

[Non-Patent Document 1] "A 24.3Me- Full Well Capacity and High Near Infrared Sensitivity CMOS Image Sensor with Lateral Overflow Integration Trench Capacitor" March 22, 2019 ITE Technical Report Vol.43, No. 11 IST2019-17 (Mar. 2019)
[Non-Patent Document 2] "A High Near-Infrared Sensitivity Over 70-dB SNR CMOS Image Sensor With Lateral Overflow Integration Trench Capacitor" IEEE TRANSACTIONS ON ELECTRON DEVICES, VOL. 67, NO. 4, APRIL 2020

### [SUMMARY OF THE INVENTION]

### [TECHNICAL PROBLEM]

In the disclosed technology of Patent Document 1, the blood vessel site is identified by light having a wavelength of 800 to 900 nm or 810 to 940 nm, which absorbs a large amount of hemoglobin, and further, light having a wavelength of 1500 to 1700 nm, which absorbs a large amount of glucose, is used to acquire data including glucose intensity data only from the blood vessel site. However, there is such a problem that when light having a wavelength of 1500 to 1700 nm is used for measuring the glucose concentration, it is affected by the absorption of light by water in the living body, and it is not possible to measure the blood glucose with high accuracy.

Further, in the disclosed technology of Patent Document 1, it is necessary to provide a first light source that irradiates light having a wavelength of 800 to 900 nm or 810 to 940 nm for identifying a blood vessel site, and a second light source that irradiates light having a wavelength of 1500 to 1700 nm for measuring glucose concentration, and alternately perform light emission by the first light source and light emission by the second light source. As described above, in the disclosed technology of Patent Document 1, since it is necessary to provide two light sources and to introduce a control unit for appropriately controlling the light emission timing of the two light sources, there is such a problem that the device becomes complicated, so that the cost for developing and controlling the device increases.

In Non-Patent Document 1 and Non-Patent Document 2, light having a wavelength of 1050 nm, which is one of the absorption peaks of glucose, is used for imaging a glucose solution, but there is a problem that the photodiode quantum efficiency at this wavelength is not always relatively high (26.7% @ 1050 nm). Further, in Non-Patent Document 1 and Non-Patent Document 2, although the glucose solution in the cell is imaged, specific measurement methods and measuring instruments for actually measuring the blood glucose in the living body in a non-invasive manner are not described.

The present invention has been made in view of the above problems, and it is the object of the present invention to provide a blood glucose measurement device capable of measuring a blood glucose in the blood while suppressing the influence of light absorption by water in a living body.

### [MEANS TO SOLVE PROBLEMS]

As a result of diligent research in view of the above problems, the present inventors found out that a minute difference in the amount of light due to the glucose concentration in the blood can be discriminated, while suppressing noise caused by absorption of light by water in the living body, by irradiating light in a specific wavelength band of near-infrared light.

More specifically, the present inventors obtained a finding that the transmittance depends on the glucose concentration in the near-infrared light wavelength band of 800 to 1100 nm, as a result of creating blood with different glucose concentration by adding glucose to the blood and performing a spectroscopic analysis.

In addition, as a result of a spectroscopic analysis of glucose-free physiological saline, the present inventors obtained a finding that there is an absorption by physiological saline in the wavelength band of 950 to 1040 nm, while, on the other hand on the other hand, there is almost no effect of absorption by physiological saline in the wavelength band of 800 to 950 nm.

Based on the above findings, the present inventors found out the usefulness of using a wavelength band of 800 to 950 nm as a wavelength for measuring glucose concentration.

The blood glucose measurement device according to the present invention comprises: a light source that irradiates light having a wavelength selected from a wavelength band of 800 to 950 nm; a sensor that receives light transmitted, reflected, or scattered in a living body and outputs information according to the amount of light received; and a blood glucose acquisition unit that acquires the blood glucose in the blood in the living body based on the information obtained by the sensor.

According to the above configuration, it is possible to provide a blood glucose measurement device capable of measuring a blood glucose in the blood while suppressing the influence of light absorption or the like due to water in the living body.

In the blood glucose measurement device according to the present invention, the sensor may be an image sensor having a plurality of pixels arranged in an array in a two-dimensional plane.

According to the above configuration, it is possible to provide a blood glucose measurement device capable of measuring a blood glucose in the blood based on the image information obtained from an image sensor.

In the blood glucose measurement device according to the present invention, an SN ratio of the sensor may be 60 dB or more.

According to the above configuration, it is possible to provide a blood glucose measurement device capable of measuring the blood glucose in the blood with high accuracy and reliability while suppressing the influence of light absorption by water in the living body.

In the blood glucose measurement device according to the present invention, a saturation charges of the sensor may be 1,000,000 or more.

According to the above configuration, it is possible to provide a blood glucose measurement device capable of measuring the blood glucose in the blood with high accuracy and reliability while suppressing the influence of light absorption by water in the living body.

In the blood glucose measurement device according to the present invention, a photodiode quantum efficiency of the sensor with respect to a near-infrared light irradiated from the light source may be 50% or more.

According to the above configuration, it is possible to provide a blood glucose measurement device capable of measuring the blood glucose in the blood with high accuracy and reliability while suppressing the influence of light absorption by water in the living body.

### [BRIEF DESCRIPTION OF DRAWINGS]

FIG. 1 is a block diagram showing an example of the configuration of a blood glucose measurement device according to an embodiment of the present invention.
FIG. 2 is a diagram showing an example of an optical sensor unit of a CMOS image sensor that can be preferably used in the embodiment of the present invention.
FIG. 3 is a schematic pixel cross-sectional view of a CMOS image sensor that can be preferably used in the embodiment of the present invention, and is a schematic cross-sectional view showing a cross section of a pixel array.
FIG. 4 is a cross-sectional view taken along the line A-A of FIG. 3, which is a schematic enlarged cross-sectional view of a pixel in and around the photodiode.
FIG. 5 is a graph showing the concentration dependence of the light transmittance of glucose.
FIG. 6 is a graph showing the wavelength dependence of the light transmittance of physiological saline.
FIG. 7 is a graph showing experimental results for evaluating the usefulness of the present invention.

### [DETAILED DESCRIPTION OF EMBODIMENTS]

Hereinafter, the embodiment of the blood glucose measurement device according to the present invention will be described with reference to the drawings.

The blood glucose measurement device according to the present invention comprises: a light source that irradiates near-infrared light; a sensor that receives light transmitted, reflected, or scattered in a living body and outputs information according to the amount of received light; and a blood glucose acquisition unit that acquires the blood glucose in the blood in the body, wherein the wavelength of the near-infrared light irradiated from the light source is selected from the wavelength band of 800 to 950 nm.

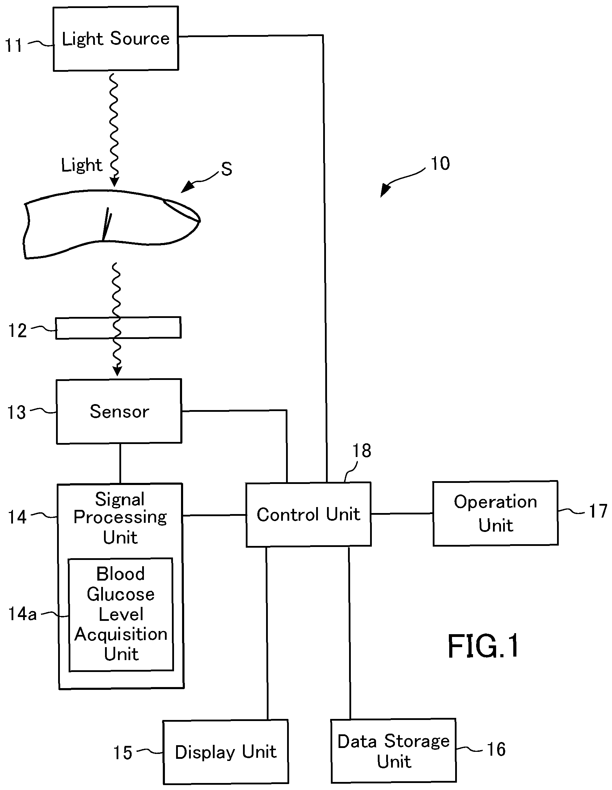

The configuration of the blood glucose measurement device in this embodiment will be described. FIG. 1 is a block diagram showing an example of the configuration of the blood glucose measurement device according to the present embodiment.

The blood glucose measurement device 10 shown in FIG. 1 includes a light source 11, a filter 12, a sensor 13, a signal processing unit 14, a display unit 15, a data storage unit 16, an operation unit 17, and a control unit 18.

The light source 11 is a device that emits light used for measuring the blood glucose of blood in a living body. The light source 11 in the present embodiment is configured to be capable of irradiating light having a wavelength suitable for measuring the blood glucose. Specifically, the light source 11 is configured to be capable of irradiating light having a wavelength selected from a wavelength band of 800 to 950 nm. The light source 11 is not particularly limited, but for example, a laser device capable of irradiating laser light can be used, and a GaAs (gallium arsenide) laser diode can be used as an example. The light source 11 in the present embodiment is preferably capable of irradiating a high amount of light (high illuminance), and for example, a laser device having a maximum output of about 500 mW may be used.

In the blood glucose measurement device 10 of the present embodiment, the subject S, which is a living body, is arranged in the irradiation direction of the light irradiated by the light source 11 at the time of measuring the blood glucose. The light irradiated to the subject S is transmitted, reflected, or scattered in the subject S.

The sensor 13 is arranged in the subject S at a position where it can receive the transmitted, reflected, or scattered light. The sensor 13 has a function of outputting an electric signal corresponding to the received light. For example, a CMOS image sensor capable of outputting an electric signal that can be finally processed as image information from the received light can constitute the image sensor 13. The CMOS sensor applicable to the present invention preferably satisfies a specific condition, and the details thereof will be described later.

A filter 12 that transmits only the light having a specific wavelength may be provided in a front stage of the light receiving portion of the sensor 13. In the present embodiment, the light source 11 is configured to be capable of irradiating light having a wavelength selected from the wavelength band of 800 to 950 nm. By providing the filter 12 that transmits only the light having the same wavelength as the light irradiated by the light source 11, only the light having the same wavelength as the light irradiated by the light source 11 may reach the sensor 13 in the rear stage. In addition, in order to further narrow down the light wavelength, the transmission characteristics of the filter 12 may be adjusted so that the light having only a part of the wavelength band included in the light irradiated by the light source 11 reaches.

The signal processing unit 14 is configured to perform a processing related to the information outputted from the sensor 13. The signal processing unit 14 has a function of performing image processing based on the information outputted from the sensor 13 and a function of outputting the image information to the display unit 15 and the data storage unit 16.

The signal processing unit 14 has a blood glucose acquisition unit 14a. The blood glucose acquisition unit 14a has a function of acquiring the blood glucose of the subject S based on the information obtained by the sensor 13. The method for acquiring the blood glucose of the subject S is not particularly limited, but, for example, the image information formed based on the information outputted by the sensor 13 is analyzed to identify the blood vessel site in the living body, thereby detecting the blood glucose (glucose concentration in blood) from the amount of light received in the blood vessel. In this case, a calibration curve showing the relationship between the amount of light received by the sensor 13 and the blood glucose may be obtained in advance, so that the blood glucose may be identified from the amount of light received at the blood vessel site with reference to the calibration curve.

The blood glucose acquisition unit 14a can acquire the blood glucose of the subject S from one image information, but the blood glucose acquisition unit 14a may acquire the blood glucose of the subject S based on a plurality of image information (for example, a plurality of frames constituting the image). For example, the blood glucose acquisition unit 14a may acquire a blood glucose from each of a plurality of image information and calculate an average value or a median value thereof.

The signal processing unit 14 has a function of outputting the blood glucose acquired by the blood glucose acquisition unit 14a to the display unit 15 and the data storage unit 16 in the same manner as the output of the image information.

The signal processing unit 14 can be realized by a processor that performs digital signal processing. An AFE (analog front end) board that adjusts the analog signal output from the sensor 13 and converts it into a digital signal and outputs it may be provided between the sensor 13 and the signal processing unit 14. Further, an analog-to-digital conversion circuit may be provided in the sensor 13 to output a digital signal from the sensor 13.

The display unit 15 is a monitor such as a liquid crystal display or an organic EL display, and has a function of displaying image information processed by the signal processing unit 14 and a blood glucose calculated by the signal processing unit 14 as visual information.

The data storage unit 16 is an auxiliary storage device such as an HDD (hard disk) or SSD (solid state disk), and has a function of storing image information and blood glucose data. Various data stored in the data storage unit 16 can be read out after the fact, and can be displayed on the display unit 15 at a desired timing or transferred to another device via a data transfer unit (not shown). The communication means at the time of data transfer may be either wired or wireless.

The operation unit 17 has a function of receiving an operation instruction from the user to the blood glucose measurement device 10. The operation unit 17 is an operation input device such as a mouse or a keyboard used by the user to input an operation instruction.

The control unit 18 has a function of controlling the operation of the blood glucose measurement device 10. As an example, the control unit 18 is connected to a light source 11, a sensor 13, a signal processing unit 14, a display unit 15, a data storage unit 16, and an operation unit 17. The control unit 18 has a function of realizing an appropriate operation in the blood glucose measurement device 10 by controlling the processing in each component unit.

The control unit 18 can be realized by a processor having a function of performing digital signal processing in the same manner as the signal processing unit 14. The signal processing unit 14 and the control unit 18 may be realized by different processors or may be realized by the same processor. The operation control in the blood glucose measurement device 10 may be realized, for example, by preparing a program described to perform a desired operation and appropriately executing the program in the signal processing unit 14 and the control unit 18.

Further, a part of the blood glucose measurement device 10 may be configured by a computer such as a personal computer. In this case, the signal processing unit 14 and the control unit 18 can be realized by a CPU that executes a program described to perform a desired operation. The display unit 15, the data storage unit 16, and the operation unit 17 can be realized by a monitor connected to a computer, an auxiliary storage device such as an HDD, a mouse, a keyboard, or the like, respectively.

As long as the light irradiated by the light source 11 is irradiated to the subject S, and the sensor 13 is configured to receive the light transmitted, reflected, or scattered in the subject S, the arrangement positions of the light source 11, the subject S and the sensor 13 are not particularly limited. For example, as schematically shown in FIG. 1, the sensor 13 may be arranged in the irradiation direction of the light irradiated by the light source 11, and the sensor 13 may directly receive the transmitted light transmitted through the subject S. Alternatively, the sensor 13 may be arranged at a position where the sensor 13 does not directly receive the light transmitted through the subject S, and the sensor 13 may mainly receive the reflected light or the scattered light from the subject S.

The subject S may be a part of the subject's living body, and for example, the subject's fingers, wrists, arms, ears, and the like can be used as the measurement site. The blood glucose measurement device 10 is configured so that the blood glucose in the blood can be acquired from the image information obtained by the imaging the blood in the living body. The blood glucose measurement device 10 may be a wearable terminal that can be worn by the subject.

Next, the sensor 13 will be described. As the sensor 13 in the present embodiment, for example, the CMOS image sensors described in Non-Patent Document 1 and Non-Patent Document 2 can be preferably used. The disclosures of Non-Patent Document 1 and Non-Patent Document 2 are incorporated herein by reference.

Non-Patent Document 1 and Non-Patent Document 2 describe CMOS image sensors in which a horizontal overflow accumulation trench capacity is mounted on each pixel on a p-type Si substrate having a low impurity concentration in which the impurity concentration is reduced to about 10¹² cm⁻³, and a high number of saturated electrons (>10 million) with high quantum efficiency in the near-infrared region is combined. This CMOS image sensor achieves a linear response to light from low to high illuminance, a saturation charge number of 24,300,000, a signal-to-noise ratio (SN ratio) of 71.3 dB, and high quantum efficiency in the wide light wavelength band of 200 to 1100 nm.

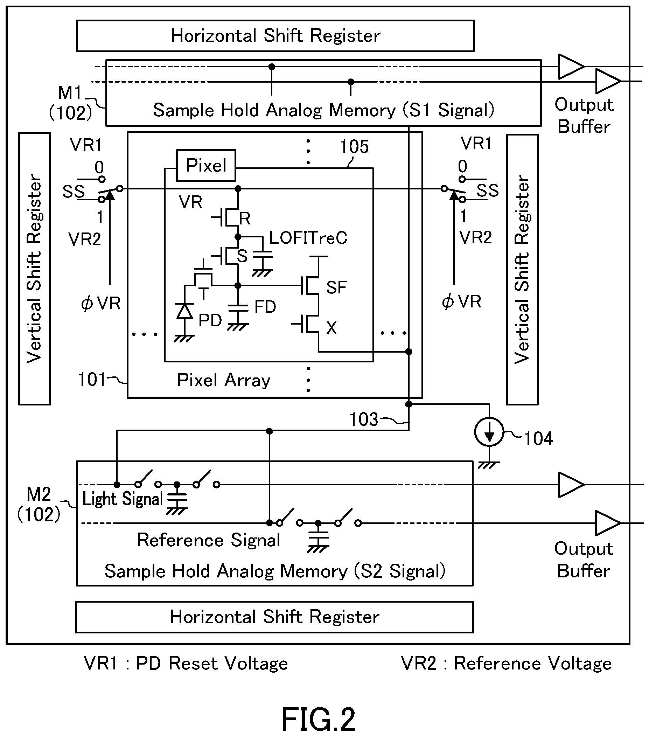

Hereinafter, the outline and features of the CMOS image sensors described in Non-Patent Document 1 and Non-Patent Document 2 will be described with reference to FIGS. 2 to 4.

FIG. 2 is a diagram showing an example of an optical sensor unit of a CMOS image sensor that can be suitably used in the present embodiment. FIG. 2 schematically shows an equivalent circuit diagram including a pixel circuit and a read circuit for one row as a part of the optical sensor unit of the CMOS image sensor.

The optical sensor unit of the CMOS image sensor shown in FIG. 2 is constituted by a pixel array unit 101 and a reading unit 102 including sample hold analog memories M1 and M2. The pixels 105 included in the pixel array unit 101 and the reading unit 102 are electrically connected to each other through the pixel train output signal line 103. A current source 104 composed of, for example, a MOS transistor is connected to the pixel train output signal line 103.

Pixel 105 is constituted by an embedded completely depleted photodiode PD, that generates an optical charge according to the intensity of light, a transfer gate T that transfers the optical charge from the photodiode PD, a floating diffusion capacitance FD in which the optical charge is transferred through the transfer gate T, a horizontal overflow storage trench capacity LOFITreC which stores the optical charge overflowing from the photodiode PD during the optical charge storage operation, a connection switch S that electrically couples or divides the floating diffusion capacitance FD and the horizontal overflow storage trench capacity LOFITreC, a reset gate R for discharging the optical charge inside the horizontal overflow storage trench capacity LOFITreC and floating diffusion capacitance FD which is formed to connect directly to the horizontal overflow storage trench capacitance LOFITreC and to the floating diffusion capacitance FD via connection switch S, a source follower amplifier SF that amplifies and converts the optical charge in the horizontal overflow storage trench capacity LOFITreC and floating diffusion capacitance FD into a voltage signal, and a pixel selection switch X for pixel selection formed to connect to the source follower amplifier SF.

In the optical sensor unit of the CMOS image sensor, a plurality of pixels 105 having the above configuration are arranged in an array in a two-dimensional plane. At both ends of the pixel array section 101, in which 105 pixels are arranged in array form, a selection switch SS is provided to control the voltage of the drive line connected to the gate electrode of the reset gate R. The system is configured so that either the PD reset voltage VR1 or the reference voltage VR2 can be selected by switching the selection switch SS with the selection pulse cpVR.

Sample hold analog memories M1 and M2 are connected to the pixel train output signal line 103.

The sample hold analog memory M1 is a train circuit unit that outputs a voltage signal in which the optical charge transferred into the floating diffusion capacitance FD is converted. The sample hold analog memory M1 is configured to output a reference signal including thermal noise taken in when the floating diffusion capacitance FD is reset to a predetermined voltage and a voltage signal in which a voltage signal based on the amount of optical charge is superimposed on the thermal noise, to remove the fixed pattern noise and thermal noise caused by variation in the characteristics of the source follower amplifier SF due to the operating amplifier outside the pixel chip, so that a high-sensitivity signal S1 that captures light emission under low illuminance can be obtained.

On the other hand, the sample hold analog memory M2 is a train circuit unit that outputs a voltage signal in which the optical charge transferred to the horizontal overflow storage trench capacity LOFITreC and the floating diffusion capacitance FD is converted. The sample hold analog memory M2 is configured to output a voltage signal and a reset level signal based on the amount of light charge, to remove the fixed pattern noise and thermal noise caused by variation in the characteristics of the source follower amplifier SF due to the operating amplifier outside the pixel chip, so that a high saturation signal S2 that captures light emission under high emission can be obtained.

Furthermore, the sample hold analog memory M2 is configured to outputs a high saturation signal S2 indicating the difference between the signal voltage level after the storage period and the reference voltage VR2, by setting the voltage level of the reference voltage VR2, which is switched by the selection switch SS as described above, to an intermediate value between the saturation level and the signal level. And M2 is configured to be able to output a high saturation signal S2 capable of capturing a minute change in the amount of light under high illuminance with high accuracy, by amplifying the signal using a gain amplifier or the like in the signal readout circuit in the subsequent stage.

The optical sensor unit of the CMOS image sensor shown in FIG. 2 can operate in two operation modes, which are an operation mode that supports a wide dynamic range (LOFIC operation mode), and an operation mode that can capture changes in the amount of light in the high illuminance region (Dual VR operation mode). In the LOFIC operation mode, the optical sensor unit of the CMOS image sensor can output the above-mentioned high-sensitivity signal S1 and high-saturation signal S2, can achieve a wide dynamic range with a single exposure, and is adaptable to luminescence imaging, which captures minute luminescence in low light conditions, and to imaging targets with large differences in brightness and darkness. On the other hand, in the Dual VR operation mode, the optical sensor portion of the CMOS image sensor is specialized for, for example, absorption imaging under high emittance, and it is possible to clearly capture minute changes in the amount of light even under high emittance.

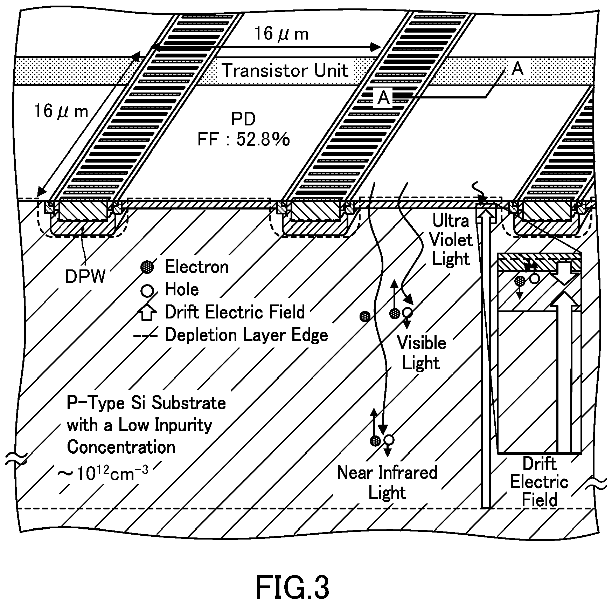

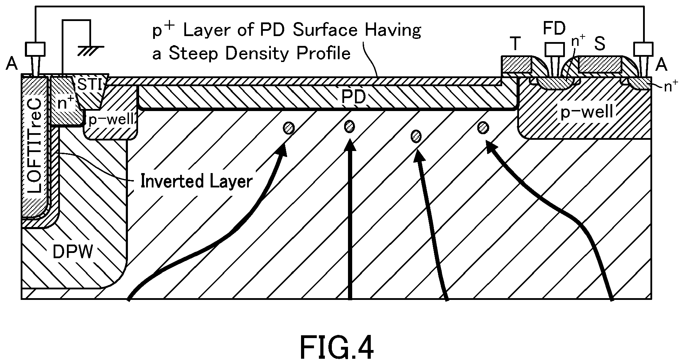

FIG. 3 is a schematic pixel cross-sectional view of a CMOS image sensor that can be suitably used in the present embodiment, and is a schematic cross-sectional view showing a cross section of a pixel array. Further, FIG. 4 is a cross-sectional view taken along the line A-A of FIG. 3, which is a schematic enlarged cross-sectional view of a pixel in and around the photodiode.

For the CMOS image sensor shown in FIGS. 3 and 4, a low impurity concentration p-type Si substrate (high resistance substrate) made of a wafer having a low impurity concentration and an extremely low oxygen concentration manufactured by the Cz (Czochralski) method is used. The CMOS image sensor is manufactured, for example, by a process of forming STI (shallow trench isolation) which is an element separation region, forming a horizontal overflow storage trench capacitance LOFITreC, forming a transistor portion and a photodiode, and forming a metal wiring.

This CMOS image sensor achieves high quantum efficiency in the wide light wavelength band of 200 to 1100 nm. This CMOS image sensor adopts a surface irradiation structure so that near-infrared light with a long wavelength does not affect the operation of the transistor in the pixel, and in addition, in order to achieve both high sensitivity and high light resistance to ultraviolet light, a p ⁺ layer having a steep density profile is formed on the surface of the photodiode PD as shown in FIG. 4.

As shown in FIG. 3, a photodiode PD and a transistor portion are arranged on the surface of the Si substrate to form a pixel array. As an example, the size of the pixel array is 2.1 mm in the horizontal direction × 2.1 mm in the vertical direction, the number of pixels is 128 in the horizontal direction × 128 in the vertical direction, the pixel size is 16 µm in the horizontal direction × 16 µm in the vertical direction, and the opening ratio is 52.8%. Further, a horizontal overflow storage trench capacity LOFITreC is provided in each pixel. The horizontal overflow storage trench capacity LOFITreC is a capacitor containing a three-dimensional structure, and it is composed of a trench (shallow groove) formed in the pixel of the Si substrate, an oxide film formed along the trench, and a doped poly-Si electrode node embedded in the trench. By accumulating the trench capacity at a high density as the horizontal overflow storage trench capacity LOFITreC, the saturation charge number can be increased so that the opening ratio can be improved.

Leakage current may occur between the embedded n-type layer of the photodiode PD and the inverted layer induced by the horizontal overflow storage trench capacity LOFITreC. As shown in FIG. 4, a deep p-well (DPW) is formed so as to cover the entire surface of the horizontal overflow storage trench capacity LOFITreC in order to suppress the generation of leakage current while maintaining the opening ratio. The concentration of DPW is optimized so as to be able to obtain a uniform capacitance over the signal voltage range of the horizontal overflow storage trench capacitance LOFITreC (For example, 0.5-3.0 V). Further, in order to keep the leak current from the charge storage node of the horizontal overflow storage trench capacity LOFITreC low, the CMOS image sensor is so configured that the charge overflowing from the photodiode PD and the floating diffusion capacitance FD is stored in the electrode node in the horizontal overflow storage trench capacity LOFITreC.

Further, near-infrared light has a large penetration depth from the surface of the photodiode PD, so that, in order to detect near-infrared light, it is necessary to accumulate and detect the optical charge photoelectrically converted in the deep portion of the photodiode PD. In order to drift the light charge due to the near-infrared light generated in the deep part of the photodiode PD to the photodiode PD, in this CMOS image sensor, a potential gradient is formed in the p-well and DPW under the region of the transistor portion. Further, the inverted layer and the n + layer induced at the Si substrate side interface of the horizontal overflow storage trench capacity LOFITreC are connected to the ground (GND).

Further, a negative potential (for example, a potential of about 3.0 V at maximum) may be applied to the back surface side (lower side of FIGS. 3 and 4) of the Si substrate to form an electric field in the depth direction of the Si substrate. Alternatively, the thickness of the Si substrate may be reduced. As a result, it is possible to suppress interpixel crosstalk of light charges due to the charge diffusion effect during drift for near-infrared light having a large penetration length, and improve the sensitivity and resolution of near-infrared light.

The CMOS image sensor described above with reference to FIGS. 2 to 4 describes the CMOS image sensor described in Non-Patent Document 1 and Non-Patent Document 2. In the present invention, the CMOS image sensors described in Non-Patent Document 1 and Non-Patent Document 2 can be preferably used. However, the present invention is not limited to the use of the CMOS image sensor described in Non-Patent Document 1 and Non-Patent Document 2, and for example, a sensor satisfying the specific conditions listed below can be applied to the present invention.

The sensor 13 applicable to the present invention is a sensor having a saturation charge number of 1,000,000 or more, preferably a saturation charge number of 3,200,000 or more, and particularly preferably a saturation charge number of 10,000,000 or more. Further, the sensor 13 applicable to the present invention has an SN ratio of 60 dB or more (corresponding to a saturation charge number of about 1,000,000 or more), particularly preferably 65 dB or more (corresponding to a saturation charge number of about 3,200,000 or more), and particularly preferably 70 dB or more (corresponding to a saturation charge number about 10,000,000 or more). A sensor having a saturation charge number or an SN ratio satisfying the above conditions is suitable because it has a high SN ratio performance and can capture a minute difference in the amount of light under high illuminance.

It is preferable that the sensor 13 according to the present invention can clearly image a fluid moving in a living body (for example, blood cells in the blood) at a high frame rate. Since a sensor having a saturation charge number or an SN ratio satisfies the above conditions can increase a signal amount per frame, it is preferable , since even when the frame rate is increased (for example, 30 fps or more), it is possible to output a clear image that captures a minute difference in the amount of light under high illuminance, with the result that it becomes possible to measure biological information with high accuracy.

Further, in order to increase the signal amount in the sensor 13, it is also important to increase the light amount of the light source 11. Therefore, in addition to the sensor having a saturation charge number of 1,000,000 or more or a sensor having an SN ratio of 60 dB or more as described above, it is preferable to use a high output light source 11 capable of emitting a high amount of light corresponding to this sensor.

As the sensor 13 according to the present invention, it is preferable to apply a sensor having a capacitor with a three-dimensional structure in the pixel and capable of accumulating light charges. The saturation charge number can be increased, by using a capacitor having a three-dimensional structure in the pixel. For the capacitor including the three-dimensional structure, for example, a silicon trench structure can be adopted, and further, the leakage current can be reduced by adopting a configuration in which the light charge is accumulated in the trench-embedded electrode node. However, the capacitor including the three-dimensional structure is not limited to the silicon trench structure, and a metal-insulating film-metal type capacitor having a three-dimensional structure formed in the wiring layer may be used.

As the sensor 13 according to the present invention, it is preferable to apply a sensor provided with a deep p-well (DPW) so as to cover the region of the capacitor including the three-dimensional structure. By this configuration, it is possible to suppress the leakage current from the capacitor including the three-dimensional structure to the photodiode and to form a potential structure for drifting the optical charge generated in the deep portion of the photodiode to the photodiode.

As the sensor 13 according to the present invention, it is preferable to apply a sensor having a capacitor having a uniform capacitance in a specific signal voltage range. The specific signal voltage range is preferably, for example, 0.5 to 3.0 V, and it is preferable that a linear response can be obtained with respect to the amount of light received in the specific signal voltage range.

As the sensor 13 according to the present invention, it is preferable to apply a sensor having a photodiode quantum efficiency of 50% or more with respect to near-infrared light (light having a wavelength of 800 to 950 nm) used in the present invention. By using a CMOS image sensor whose photodiode quantum efficiency satisfies the above conditions, the received near-infrared light is efficiently converted into electric charge, and high sensitivity to near-infrared light is realized. In order to improve the photodiode quantum efficiency, it is conceivable to stretch the depletion layer using a high resistance substrate, that is, to stretch the effective sensitivity region in the depth direction.

As the sensor 13 according to the present invention, it is preferable to apply a sensor capable of applying an electric potential to a substrate to form an electric field in the depth direction of the substrate. As a result, it is possible to suppress crosstalk between pixels of signal charges (light charges) for near-infrared light having a large penetration length and improve the resolution of near-infrared light.

Hereinafter, the features and usefulness of the blood glucose measurement device 10 according to the present invention will be described.

The present inventors have diligently studied the wavelength of near-infrared light that is effective in measuring the non-invasive blood glucose.

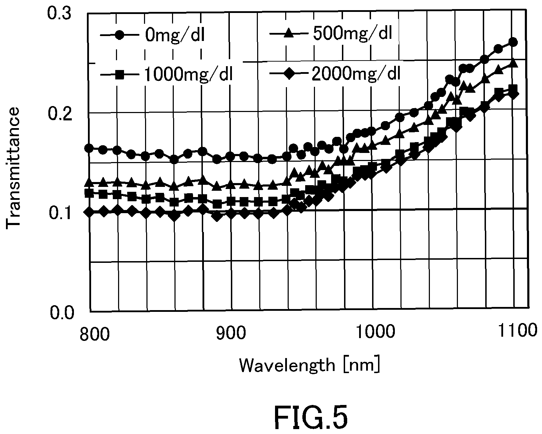

In order to investigate the concentration dependence of the light transmittance of glucose, the present inventors prepared blood having a different glucose concentration by adding glucose to the blood and performed a spectroscopic analysis. The results are shown in FIG. 5. FIG. 5 is a graph showing the concentration dependence of the light transmittance of glucose. The vertical axis (transmittance) represents the light transmittance obtained by spectroscopic analysis. The horizontal axis (wavelength) represents the wavelength of light used for the spectroscopic analysis.

FIG. 5 shows the light transmittance in the wavelength band of 800 to 1100 nm for blood prepared at glucose concentrations of 0 mg / dl, 500 mg / dl, 1000 mg / dl, and 2000 mg / dl, respectively. From the graph of FIG. 5, it can be seen that the higher the glucose concentration, the lower the transmittance of light of the same wavelength. From the above, the present inventors have found out that the light transmittance of glucose depends on the glucose concentration in the wavelength band of 800 to 1100 nm.

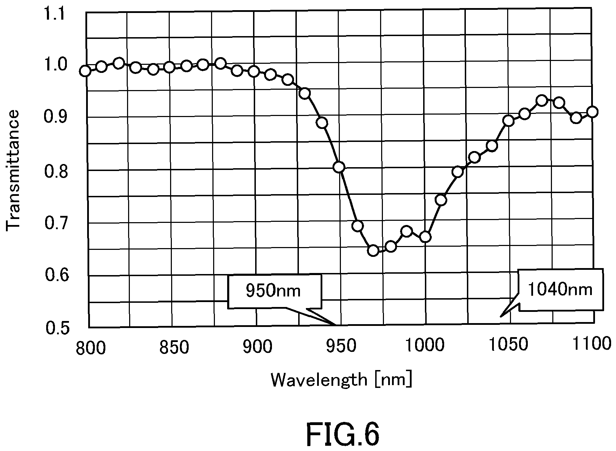

The present inventors further performed the spectroscopic analysis of the physiological saline in order to investigate the wavelength dependence of the light transmittance of the physiological saline. The results are shown in FIG.6. FIG. 6 is a graph showing the wavelength dependence of the light transmittance of physiological saline. The vertical axis (transmittance) represents the light transmittance obtained by spectroscopic analysis. The horizontal axis (wavelength) represents the wavelength of light used for the spectroscopic analysis.

From the graph of FIG. 6, it can be seen that in the wavelength band of 950 to 1040 nm, the light transmittance of the physiological saline is significantly reduced, while in the wavelength band of 800 to 950 nm, the light transmittance of the physiological saline is close to 1.0. From the above, the present inventors obtained a finding that in the wavelength band of 950 to 1040 nm, the light transmittance is greatly reduced and the influence of light absorption by the physiological saline is large, while in the wavelength band of 800 to 950 nm, there is almost no light absorption by the physiological saline. It should be noted that it has been confirmed that even when ion-exchanged water was used instead of physiological saline, the same results as in the graph of FIG. 6 were obtained, and the absorption of light in the wavelength band of 950 to 1040 nm was affected by the water molecules.

When measuring the blood glucose of the blood in the blood vessel non-invasively, the sensor receives light transmitted, reflected, or scattered by irradiating light from the outside of the skin, and the blood glucose is measured based on the amount of received light. Near-infrared light radiated from the outside of the skin toward the inside of the living body passes through the epidermis and reaches the capillaries in the dermis and the blood vessel in the subcutaneous tissue. However, body fluid is contained inside and outside of the cells constituting the epidermis and dermis, and the amount of light received by the sensor may decrease due to the influence of the absorption of light by the body fluid. For this reason, it is desirable to use light having a wavelength that is less absorbed by body fluid.

From the above spectroscopic analysis results, the present inventors have completed the present invention, based on the finding that it is effective to use light having a wavelength selected from 800 to 1100 nm to measure the glucose concentration, and further the finding that it is effective to use light having a wavelength selected from 800 to 950 nm in order to suppress the decrease in the amount of light received by the sensor, which is hardly affected by the absorption of light by physiological saline, which is the main component of body fluid. This means that the blood glucose measurement device 10 according to the present invention is characterized in that the blood glucose is measured using light having a wavelength selected from 800 to 950 nm.

It should be noted that, in the above-mentioned Patent Document 1, it is described that a wavelength (wavelength of 800 to 900 nm or 810 to 940 nm) in which the absorbance of hemoglobin is high is used in order to identify the blood vessel site. On the other hand, the light in the wavelength band of 800 to 950 nm used in the present invention is not used for identifying a blood vessel site, but is used for measuring a blood glucose in the blood.

The light in the wavelength band of 800 to 950 nm used in the present invention may be affected by the absorption of light by hemoglobin and the like. Since the measurement target in the present invention is the glucose concentration (blood glucose) in the blood and it is necessary to irradiate the blood with light, it is inevitable that the measurement will be affected by the absorption of light due to hemoglobin in the present invention as well. However, as described above, it has been confirmed that the light transmittance in the wavelength band of 800 to 950 nm depends on the glucose concentration, and the blood glucose is measured using light having the wavelength band of 800 to 950 nm, while the present invention is still affected by the absorption of light by hemoglobin to some extent.

Due to the influence of light absorption by hemoglobin, the blood vessel site may be imaged relatively darker than other sites (non-vascular site). For this reason, it is preferable to capture high illumination light using a sensor having a saturation charge number of 1,000,000 or more, so that the blood vessel site can be imaged brightly and clearly. In order to capture high illuminance light using a sensor having a saturation charge number of 1,000,000 or more, it is preferable to irradiate a living body with a high amount of light using a high output light source.

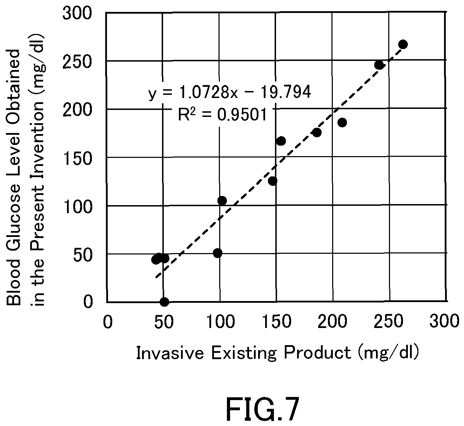

Further, in order to evaluate the effectiveness of the present invention, the present inventors conducted an experiment in which the blood having an adjusted glucose concentration was put into a cell, irradiated the cell with light to measure the blood glucose in the blood, using an LED capable of irradiating near-infrared light of 810 nm. The results of this experiment are shown in FIG. 7.

FIG. 7 is a graph showing the experimental results for evaluating the usefulness of the present invention. The vertical axis (blood glucose obtained in the present invention) represents a measured value of glucose concentration (blood glucose) in blood measured non-invasively by irradiating with near-infrared light of 810 nm. The horizontal axis (invasive existing product) represents the measured value of glucose concentration (blood glucose) in the blood by a conventional blood glucose measurement device that invasively measures the blood glucose.

The blood glucose obtained in the present invention is a blood glucose measured using light having a wavelength of 810 nm selected from the wavelength band of 800 to 950 nm, and as shown in FIG. 7, has a high correlation (coefficient of determination R² = 0.95) with respect to the blood glucose obtained by the conventional invasive blood glucose measurement device. From FIG. 7, it can be understood that the blood glucose measurement device 10 according to the present invention can measure the blood glucose with the same accuracy as the conventional invasive blood glucose measurement device.

As described above, the present invention irradiates a living body with light having a wavelength selected from the wavelength band of 800 to 950 nm, thereby making it possible to acquire the blood glucose in the blood in a living body, based on the amount of received light transmitted, reflected or scattered in a living body, and is applicable to a technology for non-invasively measuring the blood glucose in the blood in a living body using near-infrared light.

The embodiments described above are described for facilitating the understanding of the present invention, and are not described for limiting the present invention. Therefore, each element disclosed in the above-described embodiment is intended to include all design changes and equivalents belonging to the technical scope of the present invention.

### [EXPLANATION OF REFERENCE NUMERALS]

10 Glucose Measurement Device
11 Light Source
12 Filter
13 Sensor
14 Signal Processing Unit
14a Blood glucose Acquisition Unit
15 Display Unit
16 Data Storage Unit
17 Operation Unit
18 Control Unit
101 Pixel Array Unit
102 Reading Unit
103 Pixel Array Output Signal Line
104 Current Source
105 Pixel
S Subject

## Claims

1. A blood glucose measurement device, comprising:
a light source that irradiates light having a wavelength selected from a wavelength band of 800 to 950 nm;
a sensor that receives light transmitted, reflected, or scattered in a living body and outputs information according to the amount of light received; and
a blood glucose acquisition unit that acquires the blood glucose in the blood in the living body based on the information obtained by the sensor.

2. The blood glucose measurement device according to claim 1, wherein
the sensor is an image sensor having a plurality of pixels arranged in an array in a two-dimensional plane.

3. The blood glucose measurement device according to claim 1 or 2, wherein
an SN ratio of the sensor is 60 dB or more.

4. The blood glucose measurement device according to any one of claims 1 to 3, wherein
a saturation charge number of the sensor is 1,000,000 or more.

5. The blood glucose measurement device according to any one of claims 1 to 4, wherein
a photodiode quantum efficiency of the sensor with respect to a near-infrared light irradiated from the light source is 50% or more.
